(19) Europäisches Patentamt
European Patent Office
Office européen des brevets

(11) Veröffentlichungsnummer : **0 091 641**
**B1**

# (12) EUROPÄISCHE PATENTSCHRIFT

(45) Veröffentlichungstag der Patentschrift : **02.01.86**

(51) Int. Cl.⁴ : **C 07 D405/12**, A 61 K 31/415 // (C07D405/12, 233:61, 307:52)

(21) Anmeldenummer : **83103311.3**

(22) Anmeldetag : **05.04.83**

## (54) N-substituierte Imidazol-Derivate, ihre Herstellung, diese enthaltende Arzneimittel und ihre Verwendung.

(30) Priorität : **10.04.82 DE 3213509**

(43) Veröffentlichungstag der Anmeldung : **19.10.83 Patentblatt 83/42**

(45) Bekanntmachung des Hinweises auf die Patenterteilung : **02.01.86 Patentblatt 86/01**

(84) Benannte Vertragsstaaten : **AT BE CH DE FR GB LI NL**

(56) Entgegenhaltungen :
**EP-A- 0 004 561**
**DE-A- 3 100 364**
**GB-A- 1 565 966**
**US-A- 3 978 075**
**US-A- 4 226 874**

(73) Patentinhaber : **BASF Aktiengesellschaft**
**Carl-Bosch-Strasse 38**
**D-6700 Ludwigshafen (DE)**

(72) Erfinder : **Ostersehlt, Bernd, Dr.**
**Beuthener Strasse 7**
**D-6700 Ludwigshafen (DE)**
Erfinder : **Franke, Albrecht, Dr.**
**Mandelring 11**
**D-6706 Wachenheim (DE)**
Erfinder : **Frickel, Fritz-Frieder, Dr.**
**Silvanerweg 7**
**D-6705 Deidesheim (DE)**
Erfinder : **Thyes, Marco, Dr.**
**Thorwaldsenstrasse 1**
**D-6700 Ludwigshafen (DE)**
Erfinder : **Friedrich, Ludwig, Dr.**
**Nibelungenstrasse 8a**
**D-6831 Bruehl (DE)**
Erfinder : **Gries, Josef, Dr.**
**Roemerweg 43**
**D-6706 Wachenheim (DE)**
Erfinder : **Lenke, Dieter, Dr.**
**Kekuleplatz 1**
**D-6700 Ludwigshafen (DE)**

**EP 0 091 641 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Histamin-$H_2$-Antagonisten sind nützlich bei der Behandlung von Krankheitszuständen, die mit einer pathologisch gesteigerten Magensekretion einhergehen. Bisher bekannte Histamin-$H_2$-Antagonisten sind z. B. Ranitidin, Metiamid und Cimetidin (vgl. z. B. R. N. Brogden et al., « Ranitidine : A review of its pharmacology and therapeutic use in peptic ulcer disease and other allied diseases », Drugs *24* (1982), p. 267 bis 303 ; G. J. Durant und C. R. Ganellin et al., J. Med. Chem. *20,* Seite 901 (1977), ferner DE-AS 2 344 779, BE-PS 779 775, US-PS 3 975 530, US-PS 3 876 647 und US-PS 3 897 444 sowie GB-PS 1 565 966). Sie enthalten als gemeinsames Strukturelement einen basischen oder basisch substituierten Heterocyclus, der über eine eventuell durch ein S-Atom unterbrochene Alkylenkette mit einer Harnstoff- oder Thioharnstoff-äquivalenten Struktureinheit verbunden ist, die des weiteren nur einfache Alkylreste o.ä. trägt.

Die Wirksamkeit solcher Verbindungen ist in vielen Fällen nicht befriedigend. Der Erfindung lag daher die Aufgabe zugrunde, diesem Mangel abzuhelfen.

Es wurde nun gefunden, daß diese Schwäche der literaturbekannten Verbindungen behoben werden kann, wenn an eine Thioharnstoffgruppierung außer dem einen basischen oder basisch substituierten Heterocyclus noch ein weiterer basischer Heterocyclus mit einem durch eine Alkylenkette definierter Länge bestimmten Abstand gebunden ist. Dieser weitere Heterocyclus ist ein N-gebundenes Imidazol.

Gegenstand der Erfindung sind daher N-substituierte Imidazol-Derivate der Formel I

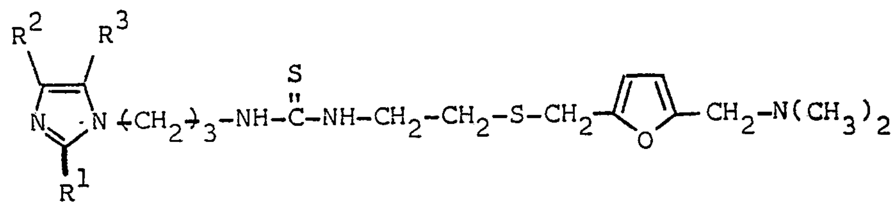

(I)

in der $R^1$ Alkyl mit 1 bis 3 C-Atomen bedeutet, $R^2$ und $R^3$ gleich oder verschieden sind und Wasserstoff oder Alkyl mit 1 bis 3 C-Atomen bedeuten, sowie deren Salze mit physiologischen Säuren.

Als erfindungsgemäß Verbindungen der Formel (I) sind beispielsweise zu nennen :

N-[3-(2-Methyl-imidazol-1-yl)propyl]-N'-{2-[[5-(N,N-dimethylaminomethyl)-2-furanyl]-methylthio]-ethyl}-thioharnstoff

2-[3-(2-Ethyl-imidazol-1-yl)propyl]-N'-{2-[[5-(N,N-dimethylaminomethyl)-2-furanyl]-methylthio]-ethyl}-thioharnstoff

N-[3-(2-Propyl-imidazol-1-yl)propyl]-N'-{2-[[5-(N,N-dimethylaminomethyl)-2-furanyl]-methylthio]-ethyl}-thioharnstoff

N-[3-(2-Isopropyl-imidazol-1-yl)propyl]-N'-{2-[[5-(N,N-dimethylaminomethyl)-2-furanyl]-methylthio]-ethyl}-thioharnstoff

N-[3-(2,4-Dimethyl-imidazol-1-yl)propyl]-N'-{2-[[5-(N,N-dimethylaminomethyl)-2-furanyl]-methylthio]-ethyl}-thioharnstoff

N-[3-(2,5-Dimethyl-imidazol-1-yl)propyl]-N'-{2-[[5-(N,N-dimethylaminomethyl)-2-furanyl]-methylthio]-ethyl}-thioharnstoff

N-[3-(2-Ethyl-4-methyl-imidazol-1-yl)propyl]-N'-{2-[[5-(N,N-dimethylaminomethyl)-2-furanyl]-methylthio]-ethyl}-thioharnstoff

N-[3-(2-Ethyl-5-methyl-imidazol-1-yl)propyl]-N'-{2-[[5-(N,N-dimethylaminomethyl)-2-furanyl]-methylthio]-ethyl}-thioharnstoff

N-[3-(2,4,5-Trimethyl-imidazol-1-yl)propyl]-N'-{2-[[5-(N,N-dimethylaminomethyl)-2-furanyl]-methylthio]-ethyl}-thioharnstoff

Als physiologisch verträgliche organische oder anorganische Säuren kommen zur Salzbildung beispielsweise in Betracht : Salzsäure, Bromwasserstoffsäure, Phosphorsäure, Schwefelsäure, Oxalsäure, Maleinsäure, Fumarsäure, Milchsäure, Weinsäure, Äpfelsäure, Zitronensäure, Salicylsäure, Adipinsäure oder Benzoesäure. Weitere Säuren können aus « Fortschritte der Arzneimittelforschung », Band 10, Seiten 224 bis 225, Birkhauser-Verlag, Basel und Stuttgart, 1966, entnommen werden.

Die Erfindung betrifft weiter ein Verfahren zur Herstellung der Verbindungen der Formel I, welches darin besteht, daß man

a) eine Thioverbindung der Formel II

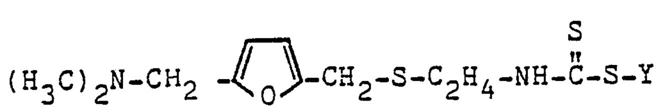

(II)

in der Y $C_1$-$C_4$-Alkyl oder Aralkyl bedeutet, mit einem ω-Aminoalkylimidazol der allgemeinen Formel III

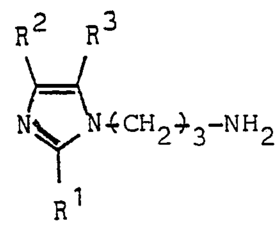

(III)

in der $R^1$, $R^2$, $R^3$ die angegebene Bedeutung haben, umsetzt oder

b) 2-[[5-(N,N-Dimethylaminomethyl)-2-furanyl]methylthio]-ethylamin mit einer Verbindung der Formel IV

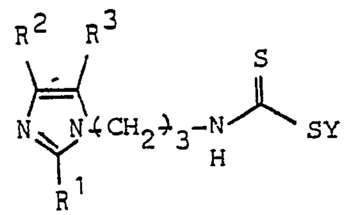

(IV)

in der $R^1$, $R^2$, $R^3$ und Y dasselbe wie oben bedeuten, umsetzt oder

c) 2-[[5-(N,N-Dimethylaminomethyl)-2-furanyl]methylthio]-ethylamin mit einer Verbindung der Formel V

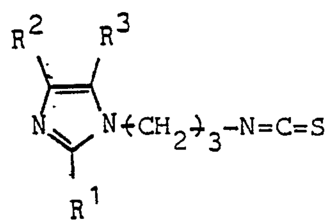

(V)

worin $R^1$, $R^2$ und $R^3$ dasselbe wie oben bedeuten, umsetzt
und die so erhaltenen Verbindungen gegebenenfalls in ihre Salze mit physiologisch verträglichen Säuren überführt.

Die Umsetzung gemäß a) wird bei Raumtemperatur oder bei höherer Temperatur, zweckmäßig bei 50 bis 120 °C, durchgeführt. Sie kann unter Atmosphärendruck oder in einem geschlossenen Gefäß unter erhöhtem Druck, gegebenenfalls unter Erwärmung auf den angegebenen Temperaturbereich, vorgenommen werden. Besonders gut gelingt sie, wenn Y eine Methylgruppe bedeutet.

Die Ausgangsverbindungen können direkt, d. h. ohne Zugabe eines Verdünnungs- oder Lösungsmittels, umgesetzt werden. Zweckmäßigerweise wird die Umsetzung jedoch in Gegenwart eines inerten Verdünnungs- oder Lösungsmittels, beispielsweise eines niederen Alkohols mit 1 bis 4 C-Atomen, wie Methanol, Ethanol oder Propanol, eines niederen gesättigten Dialkylethers, Dialkylglykolethers oder cyclischen Ethers, wie Diethylether, 1,2-Dimethoxyethylglykolether, Tetrahydrofuran oder Dioxan, eines Dialkylformamids, wie Dimethyl- oder Diethylformamid, von Dimethylsulfoxid, Acetonitril oder in Gegenwart von Wasser oder in Mischungen der genannten Lösungsmittel durchgeführt.

Am geeignetsten sind niedere Alkohole, vorzugsweise Methanol oder Ethanol sowie Wasser.

Die Umsetzung wird bevorzugt bei Temperaturen von 50 °C bis 120 °C und bei Normaldruck durchgeführt.

Die vollständige Umsetzung hängt von der Reaktionstemperatur ab und ist im allgemeinen innerhalb von 2 bis 25 Stunden beendet. Das Reaktionsprodukt kann in an sich bekannter Weise gewonnen werden, z. B. durch Filtration oder durch Abdestillieren des Verdünnungs- oder Lösungsmittels aus dem Reaktionsgemisch. Eine Reinigung der erhaltenen Verbindungen erfolgt in üblicher Weise, beispielsweise durch Umkristallisation aus einem Lösungsmittel, Überführen in das Salz einer physiologisch verträglichen Säure oder durch Säulenchromatographie.

Für das Verfahren b) der nucleophilen Substitution von SY gelten die gleichen Reaktionsmerkmale wie sie oben für die Umsetzungen von Thioverbindungen der allgemeinen Formel (II) mit Alkylaminoimidazolen der allgemeinen Formel (III) beschrieben sind.

Die Umsetzung c) wird in Gegenwart eines Lösungsmittels, beispielsweise eines niederen Alkohols mit 1 bis 4 C-Atomen, vorzugsweise Methanol, Ethanol oder Isopropanol, oder eines Dialkylformamids, wie Dimethyl- oder Diethylformamid, von Dimethylsulfoxid oder von Wasser durchgeführt. Die Reaktion erfolgt vorzugsweise bei Temperaturen von 0 bis 100 °C unter Normaldruck.

Die Gewinnung der Reaktionsprodukte kann wie bei a) beschrieben erfolgen.

Die Ausgangssubstanzen der Formeln II, III, IV, V sind literaturbekannt oder lassen sich nach bekannten Verfahren herstellen.

Schließlich betrifft die Erfindung Arzneimittel, welche neben üblichen Träger- und Verdünnungsmitteln eine Verbindung der Formel I oder ihr physiologisch verträgliches Salz als Wirkstoff enthalten.

Die erfindungsgemäßen Verbindungen können in üblicher Weise, vorzugsweise oral oder intravenös, verabfolgt werden. Die Dosierung hängt vom Alter, Zustand und Gewicht des Patienten sowie von der Applikationsart ab. In der Regel beträgt die tägliche Wirkstoffdosis zwischen etwa 0,5 und 20 mg/kg Körpergewicht bei oraler Gabe und zwischen 0,05 und 10 mg/kg Körpergewicht bei intravenöser Gabe. In besonderen Fällen kann es jedoch erforderlich sein, die Dosen zu steigern.

Die erfindungsgemäßen Verbindungen und ihre physiologisch verträglichen Säureadditionssalze weisen wertvolle pharmakologische Eigenschaften auf und sind geeignet bei der Behandlung von Krankheitszuständen, die mit einer pathologisch gesteigerten Magensekretion einhergehen, z. B. Magen- und Duodenalulcera.

Zum Nachweis der Histamin-$H_2$-Rezeptor-blockierenden Wirkung wurden folgende Methoden angewendet.

An narkotisierten (Urethan-Narkose : 1,78 g/kg intravenös = i.v.) Ratten (Stamm : Sprague-Dawley, männlich, Gewicht : 210 bis 310 g) verursacht der spezifische $H_2$-Rezeptor-Agonist Dimaprit (S-[3-(N,N-dimethylamino)-propyl]-isothioharnstoff (Literatur siehe : PARSONS, M.E. et al. Agents and Actions *7,* 31 bis 37 (1977) ; FLYNN, Sh. B. et al. : Brit. J. Pharmac. *61,* 101 bis 107 (1977)) nach i.v. Injektion von 3,16 mg Blutdrucksenkungen um durchschnittlich 27 ± 0,7 mmHg bei Ausgangsdrücken von 100 ± 1,1 mmHg (Anzahl der Tiere N = 70). $H_2$-Rezeptor-Antagonisten wie beispielsweise Cimetidin oder Ranitidin hemmen diese Dimaprit-Blutdrucksenkung spezifisch und dosisabhängig. Die Applikation der geprüften Substanzen erfolgte intravenös 5 min. vor der Dimaprit-Injektion. Als ED 50 % werden die Dosen ermittelt, welche die Dimaprit-Blutdrucksenkung um 50 % hemmen (Tab. 1).

Die Hemmung der Magensäuresekretion kommt in einem Anstieg des pH-Wertes der Magenschleimhaut zum Ausdruck. Sie wird an Gruppen von 5 wachen weiblichen Sprague-Dawley-Ratten (160 bis 180 g) untersucht. Die Tiere erhalten 48 Stunden kein Futter (Wasser ad libitum) und werden mit unterschiedlichen Dosen der Prüfsubstanzen (subcutan = s. c.) vorbehandelt. Nach 1 Stunde werden sie mit Hexobarbital-Na (46,4 mg/kg i.v.) narkotisiert. Anschließend wird eine pH-Elektrode (Philips-Spezialelektrode Typ CJP) in den Magen eingeführt und der pH-Wert auf der Oberfläche der Magenschleimhaut gemessen (pH-Wert bei unbehandelten Tieren : 1,40 ± 0,02 ; N = 200). Aus der linearen Regression zwischen den Logarithmen der applizierten Dosen und der Zunahme des pH-Wertes wird als ED 0,75 die Dosis bestimmt, die im Vergleich zu unbehandelten Kontrolltieren eine pH-Zunahme um durchschnittlich 0,75 bewirkt (Tab. 2).

Die Experimente (Tabelle 1) ergaben für die erfindungsgemäßen Verbindungen eine starke Hemmung der durch Dimaprit ausgelösten Blutdrucksenkung. Die Wirkung übertrifft die des bekannten $H_2$-Rezeptor-Antagonisten Cimetidin um den Faktor 10 bis 26. Auch im Vergleich zu Ranitidin sind die wirksamen Dosen gleich groß oder bis 2,5 mal kleiner.

Die erfindungsgemäßen Verbindungen bewirken eine Hemmung der Säuresekretion des Magens, die durch eine dosisabhängige Erhöhung des pH-Wertes auf der Oberfläche der Magenschleimhaut zum Ausdruck kommt (Tabelle 2).

Beispiel 1 übertrifft dabei die Wirkung des bekannten Pharmakons Cimetidin um den Faktor 8,7. Im Vergleich mit Ranitidin ist Beispiel 1 um den Faktor 1,7 wirksamer. Die Substanzen hemmen ferner die Bildung von Magenulcera.

Tabelle 1 : Hemmung der Dimaprit-Blutdrucksenkung

| Substanz | ED 50 %[x] | R.W.[xx] |
|---|---|---|
| Beispiel Nr. 1 | 0,092 | 23,91 |
| " " 2 | 0,10 | 22,00 |
| " " 3 | 0,17 | 12,94 |
| " " 4 | 0,22 | 10,00 |
| " " 5 | 0,084 | 26,19 |
| " " 6 | 0,22 | 10,00 |
| Ranitidin | 0,21 | 10,48 |
| Cimetidin | 2,2 | 1,00 |

Ratte, Urethan-Narkose, Applikation : i.v.

[x] ED 50 % = Dosis, welche die Dimaprit-Blutdrucksenkung um 50 % hemmt

[xx] R.W. = Relative Wirksamkeit ; Cimetidin = 1,00

Tabelle 2 : Hemmung der Magensekretion der Ratte

| Substanz | ED 0,75 % | R.W. |
|---|---|---|
| Beispiel Nr. 1 | 0,052 | 8,65 |
| Cimetidin | 0,45 | 1,00 |
| Ranitidin | 0,086 | 5,23 |

Appl. : s.c. ; R.W. = Relative Wirksamkeit

Die neuen Verbindungen können in den üblichen galenischen Applikationsformen fest oder flüssig angewendet werden, wie Tabletten, Kapseln, Pulver, Granulate, Dragees oder Lösungen. Diese werden in üblicher Weise hergestellt. Die Wirkstoffe können dabei mit den gebräuchlichen galenischen Hilfsmitteln wie Talkum, Gummiarabikum, Saccharose, Lactose, Getreide- oder Maisstärke, Kartoffelmehl, Magnesiumstearat, Alginaten, Gummi-Tragacanth, Carraghenate, Polyvinylalkohol, Polyvinylpyrrolidon, wäßrigen oder nicht wäßrigen Trägern, Netzmitteln, Dispergiermitteln, Emulgatoren und/oder Konservierungsmitteln verarbeitet werden (vgl. L.G. Goodman, A. Gilman, The Pharmacological Basis of Therapeutics). Die so erhaltenen Präparate enthalten den Wirkstoff normalerweise in einer Menge von 0,1 bis 99 Gew.%.

Beispiel 1

3,6 g 3-[1-(2-Methylimidazolyl)]-propylisothiocyanat werden in 40 ml Methanol gelöst, mit 4,3 g 2-[[5-(N,N-Dimethylaminomethyl)-2-furanyl]methylthio]ethylamin versetzt und über Nacht stehen gelassen. Anschließend wird eingedampft und der Rückstand mit Dichlormethan an Aluminiumoxid chromatographiert. Das resultierende Öl wird mit Oxalsäure kristallisiert. Man erhält 6,5 g (57 % d.Th.) N-[3-(2-Methyl-imidazol-1-yl)propyl]-N'-{2-[[5-(N,N-dimethylaminomethyl)-2-furanyl]-methylthio]-ethyl}-thioharnstoff-dioxalat vom Schmp. 126 °C.

$C_{18}H_{29}ON_5S_2 \cdot 2(COOH)_2 \times 0{,}5\ H_2O$ (585)

Ber. : 45,1 C 5,8 H 12,0 N 10,9 S

Gef. : 44,8 C 5,9 H 12,0 N 10,8 S

Beispiel 2

2,6 g N-3-(2-Isopropylimidazol-1-yl)-propyl-S-methyl-dithiocarbamat und 2,1 g 2-[[5-N,N-Dimethylaminomethyl)-2-furanyl]methyl]ethylamin werden in 100 ml Wasser 24 h bei 80 °C gerührt. Anschließend wird mit 50 ml Methanol versetzt und 6 h mit 3 g Aktivkohle gerührt. Nach Filtration wird die Lösung i. Vak. zur Trockne eingeengt. Man erhält 3,1 g (72 % d.Th.) N-[3-(2-Isopropyl-imidazol-1-yl)propyl]-N'-2-[[5-(N,N-dimethylaminomethyl)-2-furanyl]-methylthio]-ethyl-thioharnstoff als farbloses Öl.

$C_{20}H_{33}N_5S_2O$ X 0,75 $H_2O$ (438)

ber. : 55,0 C 7,9 C 16,0 N 14,6 S

gef. : 55,2 C 8,0 C 16,1 N 14,0 S

Beispiel 3

3,0 g N- 2-[[5-(N,N-Dimethylaminomethyl)-2-furanyl]methylthio]-ethyl -S-methyl-dithiocarbamat und 1,5 g 1-(3-Aminopropyl)-2-ethyl-imidazol werden in 100 ml $H_2O$ 24 h bei 80 °C gerührt. Anschließend wird eingedampft und mit Dichlormethan an Aluminiumoxid chromatographiert. Das Eluat wird eingeengt und der Rückstand mit Oxalsäure kristallisiert. Man erhält 3,5 g (59 % d. Th.) N-[3-(2-Ethyl-imidazol-1-yl)propyl]-N'- 2-[[5-(N,N-dimethylaminomethyl)-2-furanyl]-methylthio]ethyl -thioharnstoff-dioxalat vom Schmp. 137 °C.

$C_{23}H_{35}O_9N_5S_2$ (590)

ber. : 46,9 C 6.0 H 11,9 N 10,9 S

gef. : 46,7 C 5,8 H 11,7 N 11,0 S

Die in der nachfolgenden Tabelle angegebenen Verbindungen werden gemäß Beispiels 3 aus N-{2-[[5-(N,N-Dimethylaminomethyl)-2-furanyl]-methylthio}-ethyl}-S-methyl-dithiocarbamat und den entsprechenden 1-(Aminoalkyl)-imidazolen hergestellt. In allen Fällen wurden die zur Trockne eingedampften Rückstände mit Dichlormethan an Aluminiumoxid chromatographiert..

(Siehe Tabelle Seite 6 f.)

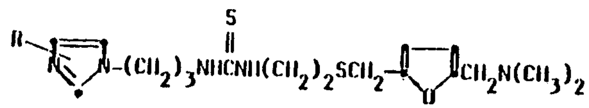

| Beispiel Nr. | R | Schmp. [°C] | Ausbeute [% d.Th.] | Salz (Hydrat-Form) | Analyse | Name |
|---|---|---|---|---|---|---|
| 4 | 2-$C_3H_7$ | 100 | 53 | $2(COOH)_2$ | 47,7 C 6,2 H 1,6 N 10,6 S<br>47,3 C 6,2 H 11,3 N 10,4 S | N-[3-(2-Propyl-imidazol-1-yl)-propyl]-N'-{2-[[5-(N,N-dimethylaminomethyl)-2-furanyl]-methylthio]-ethyl}-thioharnstoff |
| 5 | 2,4,5-tri-$CH_3$ | amorph | 31 | 0,5 $CH_3OH$<br>0,5 $H_2O$ | 54,8 C 8,0 H 15,6 N 14,3 S<br>54,3 C 7,9 H 15,5 N 14,5 S | N-[3-(2,4,5-Trimethyl-imidazol-1-yl)-propyl]-N'-{2-[[5-(N,N-dimethylaminomethyl)-2-furanyl]-methylthio]-ethyl}-thioharnstoff |
| 6 | 2-$C_2H_5$; 5-Me | 123 | 58 | $2(COOH)_2$ | 47,7 C 6,2 H 11,6 N 10,6 S<br>47,3 C 6,2 H 11,3 N 10,4 S | N-[3-(2-Ethyl-5-methyl-imidazol-1-yl)-propyl]-N'-{2-[[5-N,N-dimethylaminomethyl)-2-furanyl]-methylthio]-ethyl}-thioharnstoff |

**Patentansprüche**

1. N-substituierte Imidazol-Derivate der Formel

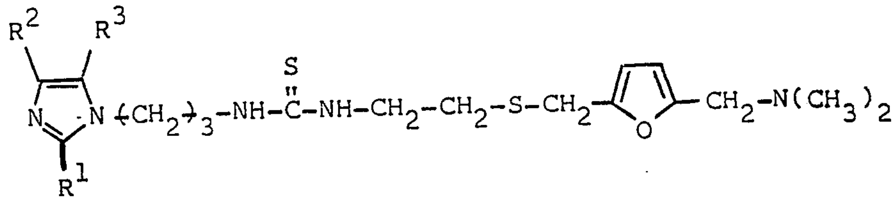

(I)

in der $R^1$ Alkyl mit 1 bis 3 C-Atomen bedeutet, und $R^2$ und $R^3$ gleich oder verschieden sind und Wasserstoff oder Alkyl mit 1 bis 3 C-Atomen bedeuten, sowie deren Salze mit physiologisch verträglichen Säuren.

2. N-[3-(2-Methyl-imidazol-1-yl)propyl]-N'-{2-[[5-(N,N-dimethylaminomethyl)-2-furanyl]-methylthio]-ethyl}-thioharnstoff.

3. N-[3-(2,4,5-Trimethyl-imidazol-1-yl)propyl}N'-{2-[[5-(N,N-dimethylaminomethyl)-2-furanyl}-methylthio]-ethyl}-thioharnstoff.

4. N-[3-(2-i-Propyl-imidazol-1-yl)propyl]-N'{2-[[5-(N,N-dimethylaminomethyl)-2-furanyl]-methylthio]-ethyl}-thioharnstoff.

5. Verfahren zur Herstellung von N-substituierten Imidazol-Derivaten der Formel I nach Anspruch 1, dadurch gekennzeichnet, daß man

a) eine Thioverbindung der Formel II

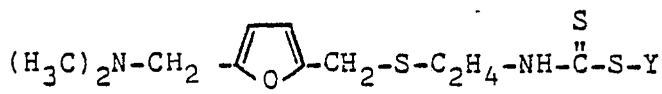

(II)

in der Y $C_1$-$C_4$-Alkyl oder Aralkyl bedeutet, mit einem Aminoalkylimidazol der algemeinen Formel III

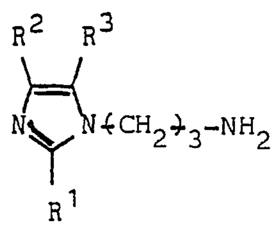

(III)

in der $R^1$, $R^2$ und $R^3$ die im Anspruch 1 angegebenen Bedeutungen haben, umsetzt oder

b) 2-[[5-(N,N-Dimethylaminomethyl)-2-furanyl]-methylthio]-ethylamin mit einer Verbindung der formel IV

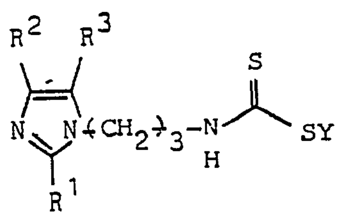

(IV)

in der $R^1$, $R^2$, $R^3$ und Y dasselbe wie oben bedeuten, umsetzt oder

c) 2-[[5-(N,N-Dimethylaminomethyl)-2-furanyl]-methyl thio]-ethylamin mit einer Verbindung der Formel V,

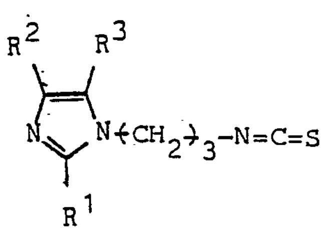

(V)

worin $R^1$, $R^2$ und $R^3$ dasselbe wie oben bedeuten, umsetzt

und die so erhaltenen Verbindungen gegebenenfalls in ihre Salze mit physiologisch verträglichen Säuren überführt.

6. Arzneimittel zur Bekämpfung von Krankheitszuständen, die mit einer gesteigerten Magensäureproduktion einhergehen, als Wirkstoff enthaltend in den üblichen Träger- und Verdünnungsmitteln eine Verbindung der Formel (I) gemäß Anspruch 1 oder eines ihrer physiologisch verträglichen Salze.

7. Verwendung eines Wirkstoffs nach Anspruch 1 zur Herstellung eines Arzneimittels gemäß Anspruch 6.

## Claims

1. An N-substituted imidazole derivative of the formula

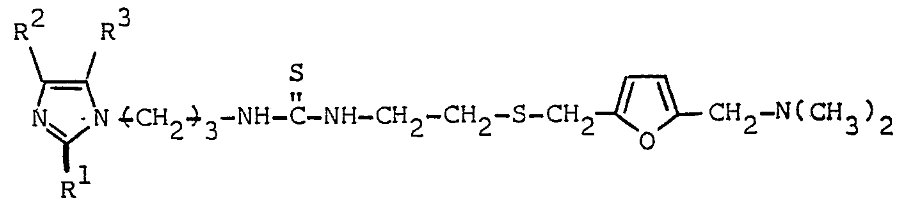

(I)

where $R^1$ is alkyl of 1 to 3 carbon atoms, and $R^2$ and $R^3$ are identical or different and are each hydrogen or alkyl of 1 to 3 carbon atoms, and its salts with physiologically tolerated acids.

2. N-[3-(2-Methylimidazol-1-yl)-propyl[-N'-{2-[[5-(N,N-dimethylaminomethyl)-furan-2-yl]-methylthio]-ethyl}-thiourea.

3. N-[3-(2,4,5-Trimethylimidazol-1-yl)-propyl]-N'-{2-[[5-(N,N-dimethylaminomethyl)-furan-2-yl]-methylthio]-ethyl}-thiourea.

4. N-[3-(2-Isopropylimidazol-1-yl)-propyl]-N'-{2-[[5-(N,N-dimethylaminomethyl)-furan-2-yl]-methylthio]-ethyl}-thiourea.

5. A process for the preparation of an N-substituted imidazole derivative of the formula I as claimed in claim 1, wherein

a) a thio compound of the formula II

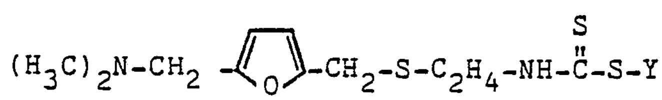

(II)

where Y is $C_1$-$C_4$-alkyl or aralkyl, is reacted with an aminoalkylimidazole of the general formula III

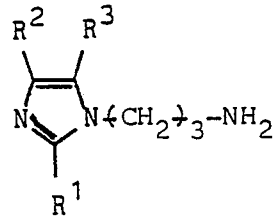

(III)

where $R^1$, $R^2$ and $R^3$ have the meanings given in claim 1, or

b) 2-[[5-(N,N-dimethylaminomethyl)-furan-2-yl]-methylthio]-ethylamine is reacted with a compound of the formula IV

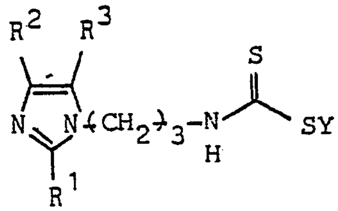

(IV)

where $R^1$, $R^2$, $R^3$ and Y have the above meanings, or

c) 2-[[5-(N,N-dimethylaminomethyl)-furan-2-yl]-methyl-thio]-ethylamine is reacted with a compound of the formula V

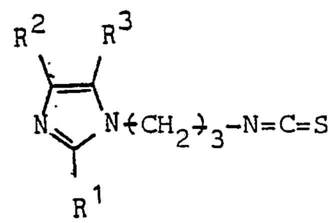

(V)

where $R^1$, $R^2$ and $R^3$ have the above meanings, and, if required, the resulting compound is converted into its salts with physiologically tolerated acids.

6. A drug for treating disorders associated with gastric hyperacidity, which contains, as the active compound, a compound of the formula (I) as claimed in claim 1, or one of its physiologically tolerated salts, in the conventional carriers and diluents.

7. The use of an active compound as claimed in claim 1 for the production of a drug as claimed in claim 6.

**Revendications**

1. Dérivés N-substitués de l'imidazole de la formule

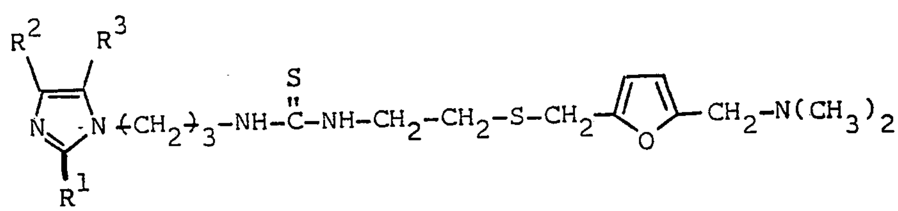

(I)

dans laquelle $R^1$ désigne un radical alkyle en $C_1$ à $C_3$ et $R^2$ et $R^3$, qui peuvent être identiques ou différents, désignent chacun un atome d'hydrogène ou un radical alkyle en $C_1$ à $C_3$, ainsi que leurs sels d'acides physiologiquement compatibles.

2. La N-[3-(2-méthyl-imidazol-1-yl)-propyl]-N'-{2-[[5-(N,N-diméthylaminométhyl)-2-furannyl]-méthylthio]-éthyl}-thio-urée.

3. La N-[3-(2,4,5-triméthyl-imidazol-1-yl)-propyl]-N'-{2-[[5-(N,N-diméthylaminométhyl)-2-furannyl]-méthylthio]-éthyl}-thio-urée.

4. La N-[3-2-isopropyl-imidazol-1-yl)-propyl]-N'-{2[[5-(N,N-diméthylaminométhyl)-2-furannyl]-méthylthio]-éthyl}-thio-urée.

5. Procédé de préparation de dérivés N-substitués de l'imidazole de la formule I selon la revendication 1, caractérisé en ce que l'on fait réagir :

a) un thio-dérivé de la formule II

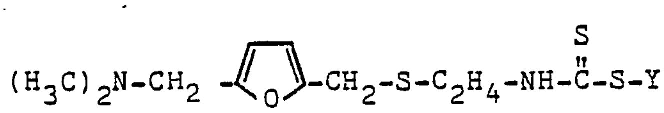

(II)

dans laquelle Y désigne un radical alkyle en $C_1$ à $C_4$ ou aralkyle, avec un amino-alkyl-imidazole de la formule générale III

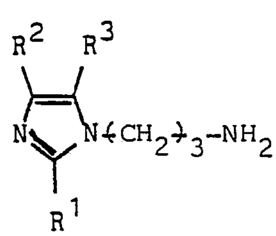

(III)

dans laquelle $R^1$, $R^2$ et $R^3$ possèdent les significations définies dans la revendication 1 ;

b) la 2-[[5-(N,N-diméthylaminométhyl)-2-furannyl]-méthylthio]-éthyl-amine avec un composé de la formule IV

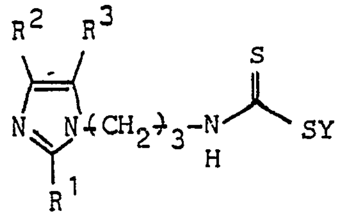

(IV)

dans laquelle $R^1$, $R^2$, $R^3$ et Y possèdent les significations définies ci-dessus, ou

c) la 2-[[5-(N,N-diméthylaminométhyl)-2-furannyl]-méthylthio]-éthyl-amine avec un composé de la formule V

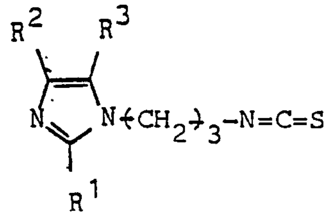

(V)

dans laquelle $R^1$, $R^2$ et $R^3$ possèdent les significations indiquées plus haut, les composés obtenus pouvant, le cas échéant, être transformés en sels d'acides physiologiquement compatibles.

6. Médicament pour lutter contre les symptômes allant de pair avec une production accrue d'acide gastrique, contenant comme principe actif un composé de la formule I selon la revendication 1 ou un sel physiologiquement compatible d'un tel composé dans des véhicules et diluants usuels.

7. Utilisation d'un composé actif selon la revendication 1 pour la préparation d'un médicament selon la revendication 6.